# EUROPÄISCHE PATENTANMELDUNG

(11) **EP 0 528 145 A1**
(43) Veröffentlichungstag der Anmeldung: **24.02.1993**
(21) Anmeldenummer: 92110889.0
(22) Anmeldetag: 26.06.1992
(51) Int. Cl.: A61B 17/58, A61B 17/16

(54) **Einführungshilfe für massive, gerade Mark- und Verriegelungsnägel**

(30) Priorität: 19.07.1991 DE 4124007
(71) Anmelder: Pennig, Dietmar, Dr. med., D-50935 Köln (DE)
(72) Erfinder: Pennig, Dietmar, Dr. med., D-50935 Köln (DE)
(74) Vertreter: Habbel, Hans-Georg, Dipl.-Ing.

(57) **Zusammenfassung**

Die Erfindung bezieht sich auf eine Einführungshilfe für geradlinige massive Mark- und Verriegelungsnägel und schlägt einerseits einen schlauchförmigen oder spiralförmigen Führungskörper (1) vor, der vorzugsweise aus einem vom Körper resorbierbaren Werkstoff besteht, zum anderen eine besondere Ausführungsform eines Nagels, der mit einer Aufnahmeöffnung (2) für einen Bohrspieß ausgerüstet ist.

## Beschreibung

Die Erfindung bezieht sich auf eine Einführungshilfe für massive, gerade Mark- und Verriegelungsnägel.

Mark- und Verriegelungsnägel sind z. B. aud der DE 25 42 263 A1 oder der DE 39 28 460 A1 bekannt.

Gerade, ungebogene und massiv ausgebildete Mark- und Verriegelungsnägel sind als Colchero-Nägel bekannt. Derartige Nägel bereiten beim Einführen in den Markraum Schwierigkeiten, da eine Einführung derartiger massiver, zum Stand der Technik gehörender Nägel an einem üblichen sogenannten Bohrspieß nicht möglich ist.

Bei den üblichen - nicht massiven - Nägeln erfolgt das Aufbohren des Markraumes nach dem Einführen eines Bohrspießes, wobei nach Entnahme des Bohrers der Bohrspieß im aufgebohrten Markraum verbleibt und dann als Führungshilfe für den einzuführenden hohlen und üblicherweise sogar einseitig offenen Nagel dient.

Das Einführen eines massiven Nagels in einen aufgebohrten Markraum ist zwar mit viel Fingerspitzengefühl und insbesondere auch unter Kontrolle unter einem Röntgenschirm möglich, aber in vielen Fällen fehlt das erforderliche Fingerspitzengefühl und in vielen weiteren Fällen steht ein solches Röntgenkontrollgerät nicht zur Verfügung.

Der Erfindung liegt daher die Aufgabe zugrunde, eine Einführungshilfe für massive Mark- und Verriegelungsnägel zu schaffen, die bewirkt, daß sich der Nagel auch im Bereich des Bruches einwandfrei von einem Knochenteil in den anderen Knochenteil führt.

Diese der Erfindung zugrundeliegende Aufgabe wird gelöst durch einen dünnwandigen, schlauchförmig ausgebildeten oder aus einer zylinderförmigen Spirale bestehenden Führungskörper mit einer Führungsöffnung zur Aufnahme eines Bohrspießes.

Vorteilhafte Ausgestaltungen sind in den Unteransprüchen erläutert.

Mit anderen Worten ausgedrückt, wird vorgeschlagen, daß - nachdem das Einführen eines Bohrspießes und das Aufbohren des Markraumes erfolgt ist - der Bohrspieß in der Markraumöffnung verbleibt und dann ein dünnwandiger schlauchförmiger oder spiralförmiger Körper an dem Bohrspieß entlang in das Innere des Markraumes eingeführt wird, und zwar über die gesamte Länge des aufgebohrten Markraumes, wobei vorzugsweise dieser schlauchförmige oder spiralartige Führungskörper aus einem vom Körper resorbierbaren Werkstoff besteht, beispielsweise wie er heute unter dem Handelsnamen "Vicryl" bzw. "Dexon" bekannt ist. Nachdem der Führungskörper in die Markraumhöhlung eingeführt ist, wird der Bohrspieß entnommen, und nunmehr führt sich der dann einzuführende massive Colchero-Nagel an dem Führungskörper bis zum untersten Ende der aufgebohrten Markraumhöhle. Gemäß einem weiteren vorteilhaften Merkmal der Erfindung kann vorgesehen sein, daß der Führungskörper selbst eine korsettstangenartige Verstärkung in seiner Wandung aufweist, so daß dadurch die Stabilität und das Standvermögen des Führungskörpers unterstützt wird.

Der Führungskörper kann aber auch aufgrund seiner Wandungskonstruktion eine ausreichende Einführungsstabilität aufweisen.

Das Einführen des Führungskörpers erfolgt entweder unter Zuhilfenahme der korsettstangenartigen Verstärkung oder unter Zuhilfenahme eines Mandrin und nach Einführen des Führungskörpers kann der Bohrspieß und der Mandrin entfernt werden, wobei die korsettstangenartige Verstärkung aus demselben vom Körper resorbierbaren Werkstoff besteht wie die Einführungshilfe, d. h. der Führungskörper selbst, oder auch aus einem anderen Werkstoff, der vor Einführen des Nagels entfernt wird.

Es wird also ein spiralförmiges oder kathederartiges Bauteil geschaffen, daß seine Aufgabe des Einführens des Colchero-Nagels erfüllt, dann aber in der Wunde verbleibt und vom Körper resorbiert wird.

Eine andere Ausführungsform kennzeichnet sich durch eine vorzugsweise im Außenumfang des Nagels angeordnete, nach außen hin offene Aufnahmeöffnung zur Aufnahme eines Bohrspießes.

Bei dieser Ausführungsform wird vorgeschlagen, daß - nachdem das Einführen eines Bohrspießes und das Aufbohren des Markraumes erfolgt ist - der Bohrspieß in der Markraumöffnung verbleibt und dann der Nagel geführt an dem Bohrspieß in die Markraumhöhlung eingesetzt wird. Hierbei führt sich also der Nagel an dem in der an seinem Außenumfang vorgesehenen Öffnung eingesetzten Bohrspieß. Der Nagel verbleibt mit dem Bohrspieß so lange in der Wunde, bis der Nagel zusammen mit dem Bohrspieß gezogen wird.

Ausführungsbeispiele der Erfindung sind in den beigefügten Zeichnungen dargestellt. Die Zeichnungen zeigen dabei in
- Fig. 1: eine schaubildliche Ansicht eines Teiles eines Führungskörpers, in
- Fig. 2: einen Schnitt gemäß der Linie 2-2 in Fig. 1, in
- Fig. 3: einen Schnitt durch einen neuen Nagel mit einer Aufnahmeöffnung für einen Bohrspieß und in
- Fig. 4: einen spiralförmigen Führungskörper.

Die Zeichnungen zeigen einen Abschnitt eines Führungskörpers 1, der an seinem unteren Ende geschlossen ist und eine Einführöffnung 2 aufweist. Im Randbereich des Führungskörpers 1 ist bei dem dargestellten Ausführungsbeispiel eine vorzugsweise entnehmbare Verstärkung 3 vorgesehen, wobei auch über den Rand verteilt mehrere solcher Verstärkungen 3 angeordnet werden können.

In Fig. 4 ist ein spiralig ausgebildeter Führungskörper dargestellt.

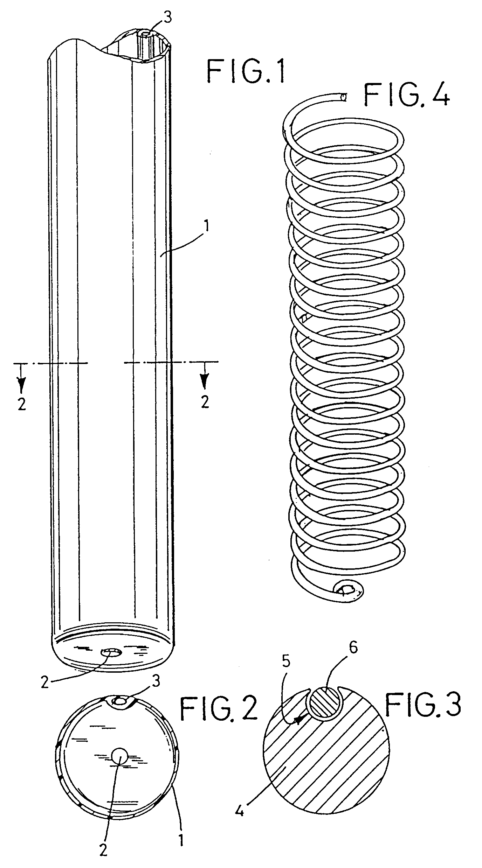

Bei dem in Fig. 3 ausgeführten Ausführungsbeispiel ist ein runder Nagel 4 dargestellt, der in seinem Randbereich eine Aufnahmeöffnung 5 aufweist, in der sich bei dem dargestellten Ausführungsbeispiel ein Bohrspieß 6 befindet, der ebenfalls natürlich nur geschnitten dargestellt ist.

Bei dieser Ausführungsform wird der Nagel 4 geführt an dem Bohrspieß 6 in die Markraumhöhlung eingesetzt und der Bohrspieß 6 verbleibt mit dem Nagel 4 so lange in der Wunde, bis der Nagel 4 zusammen mit dem Bohrspieß 6 gezogen wird. Aus diesem Grunde ist es wichtig, da auch die Öffnung 5 eine Kommunikationsöffnung nach außen hin aufweist, so daß hier keine Entzündungen entstehen können.

Die Öffnung 5 kann auch zentral im Nagel 4 angeordnet sein.

## Patentansprüche

1. Einführungshilfe für massive, gerade Mark- und Verriegelungsnägel, gekennzeichnet durch einen dünnwandigen, schlauchförmig ausgebildeten oder aus einer zylinderförmigen Spirale bestehenden Führungskörper (1) mit einer Führungsöffnung (2) zur Aufnahme eines Bohrspießes.

2. Einführungshilfe nach Anspruch 1, dadurch gekennzeichnet, daß der Führungskörper (1) aus einem vom menschlichen Körper resorbierbaren Werkstoff besteht.

3. Einführungshilfe nach Anspruch 1 oder 2, dadurch gekennzeichnet, daß der Führungskörper (1) in seiner Länge wenigstens der Länge des zu verwendenden Mark- und Verriegelungsnagels entspricht.

4. Einführungshilfe nach einem der vorhergehenden Ansprüche, dadurch gekennzeichnet, daß der Führungskörper (1) eine korsettstangenartige Verstärkung (3) seiner Wandung aufweist.

5. Einführungshilfe nach einem der vorhergehenden Ansprüche, dadurch gekennzeichnet, daß der Führungskörper (1) aufgrund seiner Wandungskonstruktion eine ausreichende Einführstabilität aufweist.

6. Einführungshilfe für einen massiven, geraden Mark- und Verriegelungsnagel, gekennzeichnet durch eine im Nagel (4) angeordnete Aufnahmeöffnung (5) zur Aufnahme eines Bohrspießes (6).

7. Einführungshilfe nach Anspruch 6, dadurch gekennzeichnet, daß die Aufnahmeöffnung (5) im Außenumfang des Nagels (4) angeordnet ist, wobei die Aufnahmeöffnung (5) nach außen hin offen ist.
